# EUROPEAN PATENT APPLICATION

(11) **EP 4 180 518 A1**
(43) Date of publication of application: **17.05.2023**
(21) Application number: 21207994.1
(22) Date of filing: 12.11.2021
(51) Int. Cl.: C12N 5/0783, A61K 35/17, A61P 35/00

(54) **T CELLS AND USES THEREOF**

(71) Applicant: Istituto Europeo di Oncologia S.r.l., 20121 Milano (MI) (IT)
(72) Inventor: Manzo, Teresa, 20121 Milano (IT); Nezi, Luigi, 20121 Milano (IT); Nava Lauson, Carina Beatriz, 20121 Milano (IT)
(74) Representative: Turri, Elisa

(57) **Abstract**

The present invention refers to a method for obtaining a T cell therapeutic comprising the steps of:
i) isolating at least one population of T cells from a biological sample isolated from a subject,
ii) activating said at least one population of T cells,
iii) treating said at least one population of T cells with a long chain fatty acids (LCFAs), preferably said long chain fatty acid being selected from: linoleic acid and oleic acid,

thereby obtaining the T cell therapeutic.

## Description

### TECHNICAL FIELD

The present invention relates to a method for obtaining a T cell therapeutic comprising the steps of:
i) isolating at least one population of T cells from a biological sample isolated from a subject,
ii) activating said at least one population of T cells,
iii) treating the activated population of T cells with a long chain fatty acid (LCFA), preferably said long chain fatty acid being selected from: linoleic acid and oleic acid,
thereby obtaining the T cell therapeutic. The present invention also relates to T cells which present an improved cell long-term functionality, metabolic fitness and a less exhaustion.

### BACKGROUND ART

Cancer immunotherapies harness the anti-tumor activity of CD8+ T cells and are a cornerstone in the treatment of cancer patients. However, the highly immunosuppressive tumor microenvironment (TME) found in solid tumors imposes metabolic constrains that restrict fitness and effector functions of CD8+ T cells. Considering the activation-induced metabolic switch is tightly intertwined with T cell activity, restoring the metabolic fitness of T cells represents a promising strategy for improving anti-tumor immunity. However, the success of this approach relies on our understanding of how metabolic programming controls T cell functions.

The present inventors have recently shown that lipid accumulation in the TME disrupts the mitochondrial dynamics of tumor-infiltrating T cells (TILs). Accumulation of long chain fatty acids (LCFAs) leads to dysregulation in T cell metabolic fitness, promoting T cell dysfunction/exhaustion and defective anti-tumors responses. Therefore, LCFAs could be considered as a molecular switch for metabolic exhaustion in intra-pancreatic CD8+ T cells showing an unexpected link between lipid metabolism dysregulation and impaired anti-tumor surveillance. While the above-mentioned study demonstrated that CD8+ T dysfunction could be associated with the accumulation of lipid metabolites, whether LCFAs can directly affect T cell activation and differentiation towards specific cell subsets by regulating metabolic fitness remains to be established. Insights on the role of LCFAs in CD8+ T cell activation and fate decision could lead to novel therapeutic approaches in which metabolic interventions can boost memory formation and anti-tumor immunity.

### SUMMARY OF THE INVENTION

Adoptive T cell therapy harnesses the immune system to recognize tumor cells and carry out an anti-tumor function. However, metabolic constraints imposed by the tumor microenvironment (TME) suppress CD8 anti-tumor responses by reshaping their metabolism landscape. In this scenario, maintaining T cells in a less-differentiated state and with high metabolic plasticity during ex vivo T cell production and after infusion may have a strong therapeutic impact. Inventors herein assessed the influence on LCFAs on T cell, particularly CD8+ T cell, activation and fate decision by modulating their mitochondrial dynamics. These insights will define new immune-metabolic pathways that could be used as a tool to boost TIL fitness and prevent metabolic T cell exhaustion. Here, inventors therefore propose a novel strategy to boost Adoptive T cell therapy efficacy by implementing T cell long-term functionality, metabolic fitness and preventing exhaustion through lipid-induced mitochondrial rewiring.

Linoleic acid (LINO or LA)-instructed T cells are endowed with higher mitochondrial fitness, potent functionality, memory-like phenotype and PD-1 down-regulation, overall evoking the ideal T cell population associated with a productive anti-tumor response. The therapeutic potential of lipid-induced metabolic rewiring in CD8 T cells was further confirmed in vivo. Adoptive T cell therapy performed with LINO-reprogrammed CD8 T cells lift higher polyfunctionality and mitochondrial function, decreased PD1 expression, ultimately resulting in improved tumor control. Importantly, LINO-induced metabolic rewiring during manufacturing of human CAR T cells, generated a CD8 T cell memory-like population empowered with higher mitochondrial fitness and coupled with a superior anti-tumor activity.

These results suggest that LINO dictate the fate of T cell, particularly CD8+ T cell, differentiation and could be considered as a molecular switch to fine-tune memory T cell formation and metabolic fitness maintenance, linking lipid metabolism to anti-tumor surveillance. This will be of fundamental importance for a new generation of adoptive T cell-based therapies.

### DETAILED DESCRIPTION OF THE INVENTION

It is an object of the present invention a method for obtaining a T cell therapeutic comprising the steps of:
i) isolating at least one population of T cells from a biological sample isolated from a subject,
ii) activating said at least one population of T cells,
iii) treating said at least one population of T cells with at least one long chain fatty acid (LCFA), preferably said long chain fatty acid being selected from: linoleic acid and oleic acid,
thereby obtaining the T cell therapeutic.

Preferably the population of T cells of step i) is CD4+, CD8+ or Gamma delta (γδ) T cells. Preferably the method further comprises a step wherein the T cell therapeutic are expanded in the presence of the at least one long chain fatty acid (LCFA), preferably said long chain fatty acid being selected from: linoleic acid and oleic acid.

In the method of the invention, preferably step ii) and iii) are performed at the same time. Preferably step ii) and/or iii) are performed for 10-15, or 10-12 days.

Preferably the obtained T cells therapeutic have at least one of the following properties: higher mitochondrial fitness, potent functionality, memory-like phenotype, higher cytotoxicity, reduced exhaustion features, increased proliferation capacity with respect to control cells and/or wherein the obtained T cell therapeutic are characterized by at least one of the following:
- increased positivity to Mitotracker,
- increase in maximal respiration levels and Spare Respiratory Capacity (SRC) and/or Oxygen Comsumption Rate (OCR) and/or proton efflux rate (PER) and/or ATP production,
- co-expression of CD44, CD62L, CD25 and CXCR3,
- an enrichment of CD127+KLRG1- population,
- increased expression of transcription factor TCF1,
- a decrease in PD1 positive cells,
- increased expression of canonical memory surface markers CD62L and/or CD127,
- increased percentage of cells producing IFNγ, TNFα and IL2,
- increase in the IFNγ+TNFα+IL2+PD1- T cell population,
- increased expression of transcription factor eomesodermin (EOMES),
- decreased levels of T-bet,
- increase in cluster size and number,
- increase in SSC values measured by flow cytometry,
- increase in CD44 percentages cell,
- increase in Blimp 1 expression,
- decreased expression of CCR7,
- decreased expression of TOX,
- increase in their pool of T central memory population, preferably at least a 1.2-fold, or a 1.3-fold, e.g. at least 1.6, increase in T central memory population, defined as CD62L+CD45RO+CD127+CD27+ and characterized by a significant decrease in PD1, with respect to controls cells.

The method of the invention preferably further comprises introducing in said population of T cells a nucleic acid sequence encoding an exogenous gene, thereby producing an engineered T cell therapeutic, preferably wherein the exogenous gene encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen.

Preferably said antigen recognizing receptor is a T cell receptor (TCR) or a chimeric antigen receptor (CAR).

Preferably said nucleic acid sequence is introduced by a vector, preferably a retroviral or lentiviral vector, and/or said nucleic acid sequence is placed at an endogenous gene locus of the T cell.

A further object of the invention is a T cell therapeutic or an engineered T cell therapeutic obtainable by the method herein disclosed.

A further object of the invention is an isolated T cell population characterized by at least one of the following:
- increased positivity to Mitotracker,
- increase in maximal respiration levels and Spare Respiratory Capacity (SRC) and/or Oxygen Comsumption Rate (OCR) and/or proton efflux rate (PER) and/or ATP production,
- co-expression of CD44, CD62L, CD25 and CXCR3,
- an enrichment of CD127+KLRG1- population,
- increased expression of transcription factor TCF1,
- a decrease in PD1 positive cells,
- increased expression of canonical memory surface markers CD62L and/or CD127,
- increased percentage of cells producing IFNγ, TNFα and IL2,
- increase in the IFNγ+TNFα+IL2+PD1- T cell population,
- increased expression of transcription factor eomesodermin (EOMES),
- decreased levels of T-bet,
- increase in cluster size and number,
- increase in SSC values measured by flow cytometry,
- increase in CD44 percentages cell,
- increase in Blimp 1 expression,
- decreased expression of CCR7,
- decreased expression of TOX,
- increase in their pool of T central memory population, preferably at least a 1.2-fold, or a 1.3-fold, e.g. at least 1.6, increase in T central memory population, defined as CD62L+CD45RO+CD127+CD27+ and characterized by a significant decrease in PD1, with respect to controls cells.

Another object of the invention is an isolated T cell characterized by being CD44+, CD62L+, CD25+, CXCR3+, CD127+ and KLRG1- and PD1-
and/or by being CD62L+CD45RO+CD127+CD27+
and/or by at least one of:

- increased positivity to Mitotracker,
- increase in maximal respiration levels and Spare Respiratory Capacity (SRC) and/or Oxygen Comsumption Rate (OCR) and/or proton efflux rate (PER) and/or ATP production,
- increased expression of transcription factor TCF1 and /or eomesodermin (EOMES) and/or Blimp1,
- being able to produce IFNγ, TNFα and IL2,
- decreased expression of T-bet and/or CCR7 and/or TOX,
- increase in cluster size,
- increase in SSC values measured by flow cytometry
   with respect to controls cells.

Preferably the isolated T cell therapeutic or T cell population or the isolated T cell (optionally engineered) of the invention presents at least one of the features described in the Table.

Preferably the isolated T cell therapeutic or T cell population or the isolated T cell (optionally engineered) of the invention is PD1- or present a decreased expression in PD1 with respect to a control cell. Preferably the isolated T cell population or the isolated T cell of the invention are engineered to comprise a nucleic acid sequence encoding an exogenous gene wherein the exogenous gene preferably encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen. Preferably said antigen recognizing receptor is a T cell receptor (TCR) or said antigen recognizing receptor is a chimeric antigen receptor (CAR). Preferably said nucleic acid sequence is introduced by a vector and/or said nucleic acid sequence is placed at an endogenous gene locus of the T cell.

A further object of the invention is a pharmaceutical composition comprising the T cell therapeutic or the engineered T cell therapeutic, the isolated T cell population, the isolated T cell or the engineered isolated T cell population or T cell of the invention or as defined herein.

Another object of the invention is the T cell therapeutic or the engineered T cell therapeutic, the isolated T cell population, the isolated T cell or the engineered isolated T cell population or T cell of or the pharmaceutical composition of the invention or as defined herein for use as a medicament, preferably for use in the treatment of tumors, more preferably of solid tumors and hematological tumors refractory to current therapies, more preferably melanoma, neuroblastoma, lymphoma and myeloma, more preferably for adoptive cell therapy.

A further object of the invention is the use of a long chain fatty acid, preferably linoleic acid or oleic acid, to increase the pool of central memory population in an isolated population of T cells and/or to obtain an isolated T cell therapeutic and/or engineered T cell therapeutic and/or T cell population and/or a T cell as defined herein.

In the present invention, the isolation of T cells, e.g. of CD8+, is preferably carried out by a negative selection, preferably with magnetic beads.

Preferably the activation step comprises stimulation of CD3 and CD28, said stimulation being preferably carried out by a CD3 agonist and a CD28 agonist, preferably the stimulation is carried out by an antibody specific for CD3 and an antibody specific for CD28. Said antibodies being preferably activating antibodies. Preferably the activation step comprises the treatment of the cells with anti-human CD3 and anti-CD28 antibodies. Preferably the activation step comprises the expansion in a medium supplemented with at least one cytokine, preferably IL2. The activation step may also be carried treating the cells with IL2 and SIINFELK peptide.

Preferably, in the methods of the invention step ii) is performed for 24 hours to 15 or 20 days, e.g. for 24-72 hours, or 48 hours, or 10-12 days. The term "treating" comprises also the term "exposing" or "contacting".

Preferably in the methods of the invention the treatment with the long chain fatty acid, preferably with LINO, comprises culturing the cells in the presence of long chain fatty acid, preferably LINO, e.g. for 10-20, or 10-12 days.

Preferably the linoleic acid is present in an amount of 50-500, more preferably 50-200 or 50-100, even more preferably 100 micromolar.

Preferably in the methods of the invention the population of T cells is contacted (or treated) with linoleic acid for 24 hours to 15 or 20 days, e.g. for 24-72 hours, or 48 hours, or 10-12 days. Preferably during this periods the cells are expanded in culture. Preferably this step of expansion is carried out after the above step ii) or iii).

The "T cell therapeutic" may be at least one T cell or at least one T cell population.

The "Engineered T cell therapeutic" may be a T cell therapeutic with an introduced nucleic acid sequence encoding an exogenous gene, preferably wherein the exogenous gene encodes an antigen-recognizing receptor, an ortho-receptor, an immunomodulatory cytokine, a chemokine receptor, a dominant negative receptor, a transcription factor able to prevent exhaustion, preferably the antigen is a tumour antigen, a self-antigen or a pathogen antigen.

Preferably the isolated population of T cells is obtained from peripheral blood, peripheral blood mononuclear cells, bone marrow, lymph nodes tissue, cord blood, thymus issue, tissue from a site of infection, ascites, pleural effusion, spleen tissue, or tumors.

In the methods of the invention steps ii) and iii) may be sequential or performed in any order or performed at the same time, preferably they are performed at the same time.

When step ii) are iii) are sequential, the at least one population of T cells of step iii) will correspond to the at least one activated one population of T cells of step ii).

Therefore, the invention also provides a method for obtaining a T cell therapeutic comprising the steps of:
i) isolating at least one population of T cells from a biological sample isolated from a subject,
ii) activating said at least one population of T cells,
iii) treating said at least one activated population of T cells with at least one long chain fatty acid (LCFA), preferably said long chain fatty acid being selected from: linoleic acid and oleic acid, thereby obtaining the T cell therapeutic.

When step ii) are iii) are performed at the same time, the method of the invention will comprise the the following steps:
i) isolating at least one population of T cells from a biological sample isolated from a subject,
ii) activating and treating with at least one long chain fatty acid (LCFA), said at least one population of T cells, preferably said long chain fatty acid being selected from: linoleic acid and oleic acid, thereby obtaining the T cell therapeutic.

Preferably in the methods of the invention the activation of step ii) comprises culturing the population of cells in a cell culture medium comprising one or more cytokines and/or ii) an anti-CD3 antibody or CD3-binding fragment thereof, and/or iii) an anti-CD28 antibody or a CD28-binding fragment thereof, B7-1 or a CD28-binding fragment thereof, or B7-2 or a CD28-binding fragment thereof, wherein the culture activates and stimulates the T cells. The cytokines are preferably selected from the group consisting of: IL-2, IL-7, and IL-15. Preferably the one or more cytokines comprise IL-2.

Preferably the concentration of the IL-2 is about 50U/mL; of IL-7 is about 10ng/mL; of IL-15 is about 5ng/mL.

Preferably the population of cells is cultured with a soluble anti-CD3 antibody and a soluble anti-CD28 antibody.

Preferably the concentration of the anti-CD3 antibody (aCD3) is about 1ug/ml.

Preferably the concentration of the anti-CD28 antibody (aCD28) is about 1ug/ml.

Preferably aCD3 and aCD28 stim is performed only at the beginning of stimulation to increase transduction efficiency, afterwards cells are kept in media with cytokines and linoleic acid. The present methods may also comprise a freezing step. Also after thawing, cells may be treated with LA. Indeed LA-treated cells expand better than control.

In the present invention controls cells are cells that are not treated with the long chain fatty acid (LCFA), preferably said long chain fatty acid being selected from: linoleic acid and oleic acid,

In a preferred embodiment the method further comprises introducing in said population of T cells a nucleic acid sequence encoding an exogenous gene, thereby producing an engineered T cell. Preferably said introduction is performed during the activation, more preferably after 48h the activation step is started.

In a preferred embodiment said biological sample is: blood and other liquid samples of biological origin, solid tissue samples, tissue cultures of cells derived therefrom and the progeny thereof, isolated cells from biological samples as i.e. PBMCs, bone marrow, cord blood, iPSC-derived T cells, more preferably blood derived cells and cells from spleen and lymph nodes.

Preferably the long chain fatty acid is linoleic or oleic acid.

Preferably the antigen is a tumor antigen, a self-antigen or a pathogen antigen.

Preferably the antigen targeted by the CAR or the TCR is CD30, GD2 and CD19 or any type of tumor antigen that can be targeted by a CAR or a TCR redirected T cells.

The T cells (or population) obtained by the method of the invention or the T cells (or population) of the invention present at least one of the following: higher mitochondrial fitness defined as increase in mitochondrial potential and mass together with an higher energetic profile (increased OCR and PER levels), potent functionality defined as the ability to simultaneously produce different effector cytokines like IFNg, TNFa, GRZB and IL2, memory-like phenotype defined as CD62L+CD45RO+CD127+CD27+, higher cytotoxicity as determined by their increased ability to kill tumour cells in an in vitro killing assay, reduced exhaustion features as demonstrated by PD-1 down-regulation, increased proliferation capacity with respect to control cells (see table 1).

Preferably the antigen recognizing receptor is exogenous.

In one embodiment, the method of the invention comprises harvesting cells from a subject and isolating a population of cells using a closed system process. In particular embodiments, cells may be isolated from any suitable fresh or frozen sources. In certain embodiments, the isolated population of (T) cells comprises peripheral blood mononuclear cells (PBMCs). The isolated population of cells is seeded to initiate cultures and T cells are activated and stimulated by contacting the cells with primary and costimulatory ligands. In particular embodiments, populations of cells comprising activated T cells are transduced with a viral vector in order to redirect the transduced cells to a particular target antigen. In certain embodiments, the cells are transduced with a viral vector encoding a CAR or engineered TCR. Transduced cells or non-transduced cells may then be cultured in growth medium to expand immune effector cells, e.g., T cells. Therefore, the methods of the invention may comprise a further step wherein the obtained T cells are expanded. Manufactured immune effector cell compositions may then be used to treat subjects in need thereof or frozen for later use.

Preferably the activation step of the present methods is performed in the presence of linoleic acid for about 48 hours, then the cells are transduced with a virus, then they are cultured for expanding for 8-10 days.

The term, "activation" or "stimulation" refers to the state of a T cell that has been sufficiently stimulated to induce detectable cellular proliferation. In particular embodiments, activation can also be associated with induced cytokine production, and detectable effector functions. The term "activated T cells" refers to, among other things, T cells that are proliferating.

In particular embodiments, T cells can be obtained from a number of sources including, but not limited to, peripheral blood, peripheral blood mononuclear cells, bone marrow, lymph nodes tissue, cord blood, thymus issue, tissue from a site of infection, ascites, pleural effusion, spleen tissue, and tumors. In one embodiment, T cells may also be obtained from a cultured T cell line, e.g., Jurkat, SupT1, etc. In particular embodiments, a population of cells comprising T cells, e.g., PBMCs, is used in the manufacturing methods contemplated herein. In other embodiments, an isolated or purified population of T cells is used in the methods contemplated herein.

The T cell manufacturing methods contemplated are particularly useful for expanding T cells modified to express high affinity T cell receptors (engineered TCRs) or chimeric antigen receptors (CARs) in a reliable and reproducible manner. In one embodiment, the T cell is genetically modified to express one ore more engineered TCRs or CARs. As used herein, T cells modified to express an engineered TCR or CAR contemplated herein may be referred to as, "antigen-specific redirected T cells".

Naturally occurring T cell receptors comprise two subunits, an a-subunit and a β- subunit, each of which is a unique protein produced by recombination event in each T cell's genome. Libraries of TCRs may be screened for their selectivity to particular target antigens. In this manner, natural TCRs, which have a high-avidity and reactivity toward target antigens may be selected, cloned, and subsequently introduced into a population of T cells used for adoptive immunotherapy.

In one embodiment, T cells are modified by introducing a polynucleotide encoding a subunit of a TCR that has the ability to form TCRs that confer specificity to T cells for tumor cells expressing a target antigen. In particular embodiments, the subunits have one or more amino acid substitutions, deletions, insertions, or modifications compared to the naturally occurring subunit, so long as the subunits retain the ability to form TCRs conferring upon transfected T cells the ability to home to target cells, and participate in immunologically-relevant cytokine signaling. The engineered TCRs preferably also bind target cells displaying the relevant tumor-associated peptide with high avidity, and optionally mediate efficient killing of target cells presenting the relevant peptide in vivo.

The nucleic acids encoding engineered TCRs are preferably isolated from their natural context in a (naturally-occurring) chromosome of a T cell, and can be incorporated into suitable vectors as described elsewhere herein. Both the nucleic acids and the vectors comprising them usefully can be transferred into a cell, which cell is preferably a T cell. The modified T cells are then able to express both chains of a TCR encoded by the transduced nucleic acid or nucleic acids. In preferred embodiments, the engineered TCR is an exogenous TCR because it is introduced into T cells that do not normally express the particular TCR. The essential aspect of the engineered TCRs is that it has high avidity for a tumor antigen presented by a major histocompatibility complex (MHC) or similar immunological component. In contrast to engineered TCRs, CARs are engineered to bind target antigens in an MHC independent manner. The protein encoded by the nucleic acids can be expressed with additional polypeptides attached to the amino-terminal or carboxyl-terminal portion of the a-chain or β-chain of a TCR so long as the attached additional polypeptide does not interfere with the ability of the a-chain or β-chain to form a functional T cell receptor and the MHC dependent antigen recognition. Antigens that are recognized by the engineered TCRs contemplated herein include, but are not limited to cancer antigens, including antigens on both hematological cancers and solid tumors. Illustrative antigens include, but are not limited to alpha folate receptor, 5T4, αv β6 integrin, BCMA, B7-H3, B7-H6, CAIX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+MAGE 1, HL A- A3 +MAGE 1 , HLA-Al+NY-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, and VEGFR2.

The methods contemplated herein include modifying T cells to express one or more CARs as contemplated herein. In various embodiments, the present invention provides T cells genetically engineered with vectors designed to express CARs that redirect cytotoxicity toward tumor cells. CARs are molecules that combine antibody-based specificity for a target antigen (e.g., tumor antigen) with a T cell receptor- activating intracellular domain to generate a chimeric protein that exhibits a specific anti- tumor cellular immune activity. As used herein, the term, "chimeric," describes being composed of parts of different proteins or DNAs from different origins. The CARs contemplated herein comprise an extracellular domain that binds to a specific target antigen (also referred to as a binding domain or antigen-specific binding domain), a transmembrane domain and an intracellular signaling domain. The main characteristic of CARs are their ability to redirect immune effector cell specificity, thereby triggering proliferation, cytokine production, phagocytosis or production of molecules that can mediate cell death of the target antigen expressing cell in a major histocompatibility (MHC) independent manner, exploiting the cell specific targeting abilities of monoclonal antibodies, soluble ligands or cell specific coreceptors. In particular embodiments, a CAR comprises an extracellular binding domain including but not limited to an antibody or antigen binding fragment thereof, a tethered ligand, or the extracellular domain of a coreceptor, that specifically binds a target antigen that is a tumor-associated antigen (TAA) or a tumor-specific antigen (TSA). In certain embodiments, the TAA or TSA is expressed on a blood cancer cell. In another embodiment, the TAA or TSA is expressed on a cell of a solid tumor. In particular embodiments, the solid tumor is a glioblastoma, a non-small cell lung cancer, a lung cancer other than a non-small cell lung cancer, breast cancer, prostate cancer, pancreatic cancer, liver cancer, colon cancer, stomach cancer, a cancer of the spleen, skin cancer, a brain cancer other than a glioblastoma, a kidney cancer, a thyroid cancer, or the like. In particular embodiments, the TAA or TSA is selected from the group consisting of alpha folate receptor, 5T4, αv β6 integrin, BCMA, B7-H3, B7-H6, CAIX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HL A- A 1 +M AGE 1 , HLA-A2+M AGE 1 , HLA-A3+MAGE 1 , HLA-Al+NY-ESO- 1, HLA-A2+NY-ESO- 1 , HLA-A3+ Y-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, and VEGFR2. In particular embodiments, CARs contemplated herein comprise an extracellular binding domain that specifically binds to a target polypeptide, e.g, target antigen, expressed on tumor cell. As used herein, the terms, "binding domain," "extracellular domain," "extracellular binding domain," "antigen-specific binding domain," and "extracellular antigen specific binding domain," are used interchangeably and provide a CAR with the ability to specifically bind to the target antigen of interest. A binding domain may comprise any protein, polypeptide, oligopeptide, or peptide that possesses the ability to specifically recognize and bind to a biological molecule (e.g., a cell surface receptor or tumor protein, lipid, polysaccharide, or other cell surface target molecule, or component thereof). A binding domain includes any naturally occurring, synthetic, semi-synthetic, or recombinantly produced binding partner for a biological molecule of interest. In particular embodiments, the extracellular binding domain of a CAR comprises an antibody or antigen binding fragment thereof. An "antibody" refers to a binding agent that is a polypeptide comprising at least a light chain or heavy chain immunoglobulin variable region which specifically recognizes and binds an epitope of a target antigen, such as a peptide, lipid, polysaccharide, or nucleic acid containing an antigenic determinant, such as those recognized by an immune cell. Antibodies include antigen binding fragments thereof. The term also includes genetically engineered forms such as chimeric antibodies (for example, humanized murine antibodies), heteroconjugate antibodies (such as, bispecific antibodies) and antigen binding fragments thereof. See also, Pierce Catalog and Handbook, 1994-1995 (Pierce Chemical Co., Rockford, IL); Kuby, J., Immunology, 3rd Ed., W. H. Freeman & Co., New York, 1997. In particular embodiments, the target antigen is an epitope of an alpha folate receptor, 5T4, αv β6 integrin, BCMA, B7-H3, B7-H6, CAIX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+M AGE 1 , HLA-A3+MAGE1, HLA-Al+NY-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide. Light and heavy chain variable regions contain a "framework" region interrupted by three hypervariable regions, also called "complementarity-determining regions" or "CDRs." The CDRs can be defined or identified by conventional methods, such as by sequence according to Kabat et al (Wu, TT and Kabat, E. A., J Exp Med. 132(2):211-50, (1970); Borden, P. and Kabat E. A., PNAS, 84: 2440-2443 (1987); (see, Kabat et al, Sequences of Proteins of Immunological Interest, U.S. Department of Health and Human Services, 1991, which is hereby incorporated by reference), or by structure according to Chothia et al (Chothia, C. and Lesk, A.M., J Mol. Biol, 196(4): 901-917 (1987), Chothia, C. et al, Nature, 342: 877 - 883 (1989)).

The sequences of the framework regions of different light or heavy chains are relatively conserved within a species, such as humans. The framework region of an antibody, that is the combined framework regions of the constituent light and heavy chains, serves to position and align the CDRs in three-dimensional space. The CDRs are primarily responsible for binding to an epitope of an antigen. The CDRs of each chain are typically referred to as CDR1, CDR2, and CDR3, numbered sequentially starting from the N- terminus, and are also typically identified by the chain in which the particular CDR is located. Thus, the CDRs located in the variable domain of the heavy chain of the antibody are referred to as CDRH1, CDRH2, and CDRH3, whereas the CDRs located in the variable domain of the light chain of the antibody are referred to as CDRL1, CDRL2, and CDRL3. Antibodies with different specificities (i.e., different combining sites for different antigens) have different CDRs. Although it is the CDRs that vary from antibody to antibody, only a limited number of amino acid positions within the CDRs are directly involved in antigen binding. These positions within the CDRs are called specificity determining residues (SDRs). References to "VH" or "VH" refer to the variable region of an immunoglobulin heavy chain, including that of an antibody, Fv, scFv, dsFv, Fab, or other antibody fragment as disclosed herein. References to "VL" or "VL" refer to the variable region of an immunoglobulin light chain, including that of an antibody, Fv, scFv, dsFv, Fab, or other antibody fragment as disclosed herein. A "monoclonal antibody" is an antibody produced by a single clone of B lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells. Monoclonal antibodies include humanized monoclonal antibodies. A "chimeric antibody" has framework residues from one species, such as human, and CDRs (which generally confer antigen binding) from another species, such as a mouse. In particular preferred embodiments, a CAR contemplated herein comprises antigen- specific binding domain that is a chimeric antibody or antigen binding fragment thereof. In certain preferred embodiments, the antibody is a humanized antibody (such as a humanized monoclonal antibody) that specifically binds to a surface protein on a tumor cell. A "humanized" antibody is an immunoglobulin including a human framework region and one or more CDRs from a non-human (for example a mouse, rat, or synthetic) immunoglobulin. Humanized antibodies can be constructed by means of genetic engineering (see for example, U.S. Patent No. 5,585,089). In particular embodiments, the extracellular binding domain of a CAR comprises an antibody or antigen binding fragment thereof, including but not limited to a Camel Ig (a camelid antibody (VHH)), Ig NAR, Fab fragments, Fab' fragments, F(ab)'2 fragments, F(ab)'3 fragments, Fv, single chain Fv antibody ("scFv"), bis-scFv, (scFv)2, minibody, diabody, triabody, tetrabody, disulfide stabilized Fv protein ("dsFv"), and single-domain antibody (sdAb, Nanobody). "Camel Ig" or "camelid VHH" as used herein refers to the smallest known antigen- binding unit of a heavy chain antibody (Koch-Nolte, et al, FASEB J., 21 : 3490-3498 (2007)). A "heavy chain antibody" or a "camelid antibody" refers to an antibody that contains two VH domains and no light chains (Riechmann L. et al, J. Immunol. Methods 231 :25-38 (1999); WO94/04678; WO94/25591; U.S. Patent No. 6,005,079). "IgNAR" of "immunoglobulin new antigen receptor" refers to class of antibodies from the shark immune repertoire that consist of homodimers of one variable new antigen receptor (VNAR) domain and five constant new antigen receptor (CNAR) domains. Papain digestion of antibodies produces two identical antigen-binding fragments, called "Fab" fragments, each with a single antigen-binding site, and a residual "Fc" fragment, whose name reflects its ability to crystallize readily. The Fab fragment contains the heavy- and light-chain variable domains and also contains the constant domain of the light chain and the first constant domain (CHI) of the heavy chain. Fab' fragments differ from Fab fragments by the addition of a few residues at the carboxy terminus of the heavy chain CHI domain including one or more cysteines from the antibody hinge region. Fab'- SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known. "Fv" is the minimum antibody fragment which contains a complete antigen-binding site. In a single-chain Fv (scFv) species, one heavy- and one light-chain variable domain can be covalently linked by a flexible peptide linker such that the light and heavy chains can associate in a "dimeric" structure analogous to that in a two-chain Fv species. The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 404,097; WO 1993/01161; Hudson et al, Nat. Med. 9:129-134 (2003); and Hollinger et al, PNAS USA 90: 6444-6448 (1993). Triabodies and tetrabodies are also described in Hudson et al, Nat. Med. 9:129-134 (2003). "Single domain antibody" or "sdAb" or "nanobody" refers to an antibody fragment that consists of the variable region of an antibody heavy chain (VH domain) or the variable region of an antibody light chain (VL domain) (Holt, L., et al, Trends in Biotechnology, 21(11): 484-490). "Single-chain Fv" or "scFv" antibody fragments comprise the VH and VL domains of antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. For a review of scFv, see, e.g. , Pluckthun, in The Pharmacology of Monoclonal Antibodies, vol. 113, Rosenburg and Moore eds., (Springer- Verlag, New York, 1994), pp. 269-315. In a certain embodiment, the scFv binds an alpha folate receptor, 5T4, αv β6 integrin, BCMA, B7-H3, B7-H6, CALX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+M AGE 1 , HLA-A3+MAGE1, HLA-A1+ Y-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide. In certain embodiments, the CARs contemplated herein may comprise linker residues between the various domains, e.g., between VH and VL domains, added for appropriate spacing and conformation of the molecule. CARs contemplated herein, may comprise one, two, three, four, or five or more linkers. In particular embodiments, the length of a linker is about 1 to about 25 amino acids, about 5 to about 20 amino acids, or about 10 to about 20 amino acids, or any intervening length of amino acids. In some embodiments, the linker is 1, 2, 3, 4, 5, 6, 7, 8, 9, 10, 11, 12, 13, 14, 15, 16, 17, 18, 19, 20, 21, 22, 23, 24, 25, or more amino acids long. In particular embodiments a CAR comprises a scFV that further comprises a variable region linking sequence. A "variable region linking sequence," is an amino acid sequence that connects a heavy chain variable region to a light chain variable region and provides a spacer function compatible with interaction of the two sub-binding domains so that the resulting polypeptide retains a specific binding affinity to the same target molecule as an antibody that comprises the same light and heavy chain variable regions. In one embodiment, the variable region linking sequence is 1, 2, 3, 4, 5, 6, 7, 8, 9, 10, 11 , 12, 13, 14, 15, 16, 17, 18, 19, 20, 21, 22, 23, 24, 25, or more amino acids long. In a particular embodiment, the variable region linking sequence comprises a glycine-serine polymer (Gi_sSi_5)n, where n is an integer of at least 1, 2, 3, 4, or 5. In another embodiment, the variable region linking sequence comprises a (G4 S)3 amino acid linker. In particular embodiments, the binding domain of the CAR is followed by one or more "spacer domains," which refers to the region that moves the antigen binding domain away from the effector cell surface to enable proper cell/cell contact, antigen binding and activation (Patel et al, Gene Therapy, 1999; 6: 412-419). The spacer domain may be derived either from a natural, synthetic, semi-synthetic, or recombinant source . In certain embodiments, a spacer domain is a portion of an immunoglobulin, including, but not limited to, one or more heavy chain constant regions, e.g., CH2 and CH3. The spacer domain can include the amino acid sequence of a naturally occurring immunoglobulin hinge region or an altered immunoglobulin hinge region. In one embodiment, the spacer domain comprises the CH2 and CH3 of IgGl . The binding domain of the CAR is generally followed by one or more "hinge domains," which plays a role in positioning the antigen binding domain away from the effector cell surface to enable proper cell/cell contact, antigen binding and activation. A CAR generally comprises one or more hinge domains between the binding domain and the transmembrane domain (TM). The hinge domain may be derived either from a natural, synthetic, semi-synthetic, or recombinant source. The hinge domain can include the amino acid sequence of a naturally occurring immunoglobulin hinge region or an altered immunoglobulin hinge region. Illustrative hinge domains suitable for use in the CARs described herein include the hinge region derived from the extracellular regions of type 1 membrane proteins such as CD8a, CD4, CD28 and CD7, which may be wild-type hinge regions from these molecules or may be altered. In another embodiment, the hinge domain comprises a CD8a hinge region. The "transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. Illustrative TM domains may be derived from (i.e., comprise at least the transmembrane region(s) of) the alpha, beta or zeta chain of the T-cell receptor, CD3 epsilon, CD3 zeta, CD4, CD5, CD9, CD 16, CD22, CD27, CD28, CD33, CD37, CD45, CD64, CD80, CD86, CD 134, CD137, and CD 154. In one embodiment, the CARs contemplated herein comprise a TM domain derived from CD8a. In another embodiment, a CAR contemplated herein comprises a TM domain derived from CD8a and a short oligo- or polypeptide linker, preferably between 1, 2, 3, 4, 5, 6, 7, 8, 9, or 10 amino acids in length that links the TM domain and the intracellular signaling domain of the CAR. A glycine-serine linker provides a particularly suitable linker. In particular embodiments, CARs contemplated herein comprise an intracellular signaling domain. An "intracellular signaling domain," refers to the part of a CAR that participates in transducing the message of effective CAR binding to a target antigen into the interior of the immune effector cell to elicit effector cell function, e.g., activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of the cell. Effector function of the T cell, for example, may be cytolytic activity or help or activity including the secretion of a cytokine. Thus, the term "intracellular signaling domain" refers to the portion of a protein which transduces the effector function signal and that directs the cell to perform a specialized function. While usually the entire intracellular signaling domain can be employed, in many cases it is not necessary to use the entire domain. To the extent that a truncated portion of an intracellular signaling domain is used, such truncated portion may be used in place of the entire domain as long as it transduces the effector function signal. The term intracellular signaling domain is meant to include any truncated portion of the intracellular signaling domain sufficient to transducing effector function signal. It is known that signals generated through the TCR alone are insufficient for full activation of the T cell and that a secondary or costimulatory signal is also required. Thus, T cell activation can be said to be mediated by two distinct classes of intracellular signaling domains: primary signaling domains that initiate antigen-dependent primary activation through the TCR (e.g., a TCR/CD3 complex) and costimulatory signaling domains that act in an antigen-independent manner to provide a secondary or costimulatory signal. In preferred embodiments, a CAR contemplated herein comprises an intracellular signaling domain that comprises one or more "costimulatory signaling domain" and a "primary signaling domain." Primary signaling domains regulate primary activation of the TCR complex either in a stimulatory way, or in an inhibitory way. Primary signaling domains that act in a stimulatory manner may contain signaling motifs which are known as immunoreceptor tyrosine-based activation motifs or ITAMs. Illustrative examples of ITAM containing primary signaling domains that are of particular use in the invention include those derived from TCRζ, FcRy, FcRp, CD3y, CD35, CD3s, CD3^ CD22, CD79a, CD79b, and CD66d. In particular preferred embodiments, a CAR comprises a CD3ζ primary signaling domain and one or more costimulatory signaling domains. The intracellular primary signaling and costimulatory signaling domains may be linked in any order in tandem to the carboxyl terminus of the transmembrane domain. CARs contemplated herein comprise one or more costimulatory signaling domains to enhance the efficacy and expansion of T cells expressing CAR receptors. As used herein, the term, "costimulatory signaling domain," or "costimulatory domain", refers to an intracellular signaling domain of a costimulatory molecule. Illustrative examples of such costimulatory molecules include CD27, CD28, 4-1BB (CD137), OX40 (CD134), CD30, CD40, PD-1, ICOS (CD278), CTLA4, LFA-1, CD2, CD7, LIGHT, TRIM, LCK3, SLAM, DAP10, LAG3, HVEM and NKD2C, and CD83. In one embodiment, a CAR comprises one or more costimulatory signaling domains selected from the group consisting of CD28, CD137, and CD134, and a CD3ζ primary signaling domain. In one embodiment, a CAR comprises an scFv that binds an alpha folate receptor, 5T4, αv β6 integrin, BCMA, B7-H3, B7-H6, CALX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+M AGE 1 , HLA-A3+MAGE1, HLA-Al+NY-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide; a transmembrane domain derived from a polypeptide selected from the group consisting of: CD8a; CD4, CD45, PD1, and CD152; and one or more intracellular costimulatory signaling domains selected from the group consisting of: CD28, CD54, CD134, CD137, CD152, CD273, CD274, and CD278; and a CD3ζ primary signaling domain. In another embodiment, a CAR comprises an scFv that binds an alpha folate receptor, 5T4, αv β6 integrin, BCMA, B7-H3, B7-H6, CALX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+M AGE 1 , HLA-A3+MAGE1, HLA-Al+NY-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide; a hinge domain selected from the group consisting of: IgGl hinge/CH2/CH3 and CD8a, and CD8a; a transmembrane domain derived from a polypeptide selected from the group consisting of: CD8a; CD4, CD45, PD1, and CD152; and one or more intracellular costimulatory signaling domains selected from the group consisting of: CD28, CD 134, and CD 137; and a CD3ζ primary signaling domain. In yet another embodiment, a CAR comprises an scFv, further comprising a linker, that binds an alpha folate receptor, 5T4, αv β6 integrin, BCMA, B7-H3, B7-H6, CAIX, CD 19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA-A 1 +M AGE 1 , HL A- A2+M AGE 1 , HLA- A3+MAGE1, HLA-Al+NY-ESO-1, HLA-A2+ Y-ESO- 1 , HLA-A3+NY-ESO-1, IL- 1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide; a hinge domain selected from the group consisting of: IgGl hinge/CH2/CH3 and CD8a, and CD8a; a transmembrane domain comprising a TM domain derived from a polypeptide selected from the group consisting of: CD8a; CD4, CD45, PD1, and CD 152, and a short oligo- or polypeptide linker, preferably between 1, 2, 3, 4, 5, 6, 7, 8, 9, or 10 amino acids in length that links the TM domain to the intracellular signaling domain of the CAR; and one or more intracellular costimulatory signaling domains selected from the group consisting of: CD28, CD 134, and CD 137; and a CD3ζ primary signaling domain. In a particular embodiment, a CAR comprises an scFv that binds an alpha folate receptor, 5T4, αv β6 integrin, BCMA, B7-H3, B7-H6, CAIX, CD19, CD20, CD22, CD30, CD33, CD44, CD44v6, CD44v7/8, CD70, CD79a, CD79b, CD123, CD138, CD171, CEA, CSPG4, EGFR, EGFR family including ErbB2 (HER2), EGFRvIII, EGP2, EGP40, EPCAM, EphA2, EpCAM, FAP, fetal AchR, FRa, GD2, GD3,^{∗} Glypican-3 (GPC3), HLA- A1+MAGE1, HLA-A2+M AGE 1 , HLA-A3+MAGE1, HLA-Al+NY-ESO-1, HLA- A2+NY-ESO-1, HLA-A3+NY-ESO- 1 , IL-1 IRa, IL-13Ra2, Lambda, Lewis-Y, Kappa, Mesothelin, Mucl, Mucl6, NCAM, NKG2D Ligands, NY-ESO-1, PRAME, PSCA, PSMA, ROR1, SSX, Survivin, TAG72, TEMs, or VEGFR2 polypeptide; a hinge domain comprising a CD8a polypeptide; a CD8a transmembrane domain comprising a polypeptide linker of about 3 amino acids; one or more intracellular costimulatory signaling domains selected from the group consisting of: CD28, CD 134, and CD 137; and a CD3ζ primary signaling domain.

The population of cells of step ii) may be cultured for about 1 to about 48 hours prior to transduction or may be transduced at the time of activation.

The population of cells of step ii) is preferably transduced with a retroviral or lentiviral vector., preferably wherein about 1 × 10⁹ TU to about 2 × 10 ⁹ TU of viral vector are used to transduce the 1 x 10 ⁸ seeded cells.

The method may further comprises recovering the T cell therapeutic, e.g. by concentrating and washing the expanded cells.

Vectors may be non-viral or viral, such as plasmids, chromosomes, artificial chromosomes and viruses. The vector may be single stranded or double stranded. It may be linear and optionally the vector comprises one or more homology arms. The vector may also be, for example, a naked nucleic acid (e.g. DNA). In its simplest form, the vector may itself be a nucleotide of interest. The vectors used in the invention may be, for example, plasmid or virus vectors and may include a promoter for the expression of a polynucleotide and optionally a regulator of the promoter. Vectors comprising polynucleotides used in the invention may be introduced into cells using a variety of techniques known in the art, such as transformation, transfection and transduction. Several techniques are known in the art, for example transduction with recombinant viral vectors, such as retroviral, lentiviral, adenoviral, adeno-associated viral, baculoviral and herpes simplex viral vectors, Sleeping Beauty vectors; direct injection of nucleic acids and biolistic transformation.

Non-viral delivery systems include but are not limited to DNA transfection methods. Here, transfection includes a process using a non-viral vector to deliver a gene to a target cell. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology 1996 14; 556) and combinations thereof.

The term "transfection" is to be understood as encompassing the delivery of polynucleotides to cells by both viral and non-viral delivery.

Transposable elements are non-viral gene delivery vehicles found ubiquitously in nature. Transposon-based vectors have the capacity of stable genomic integration and long-lasting expression of transgene constructs in cells.

The present invention will be described by means of non-limiting examples referring to the following figures:

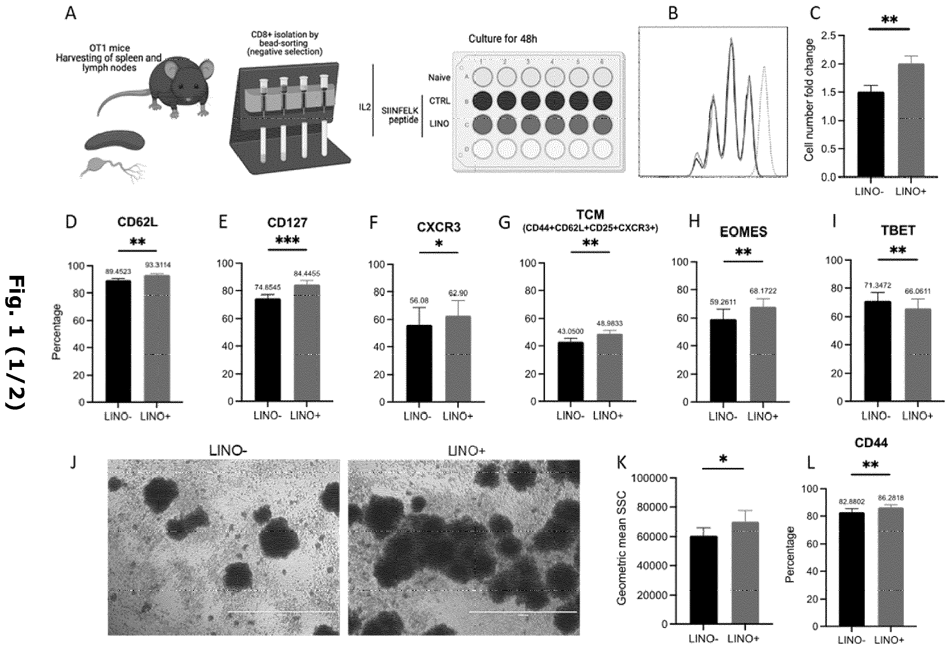

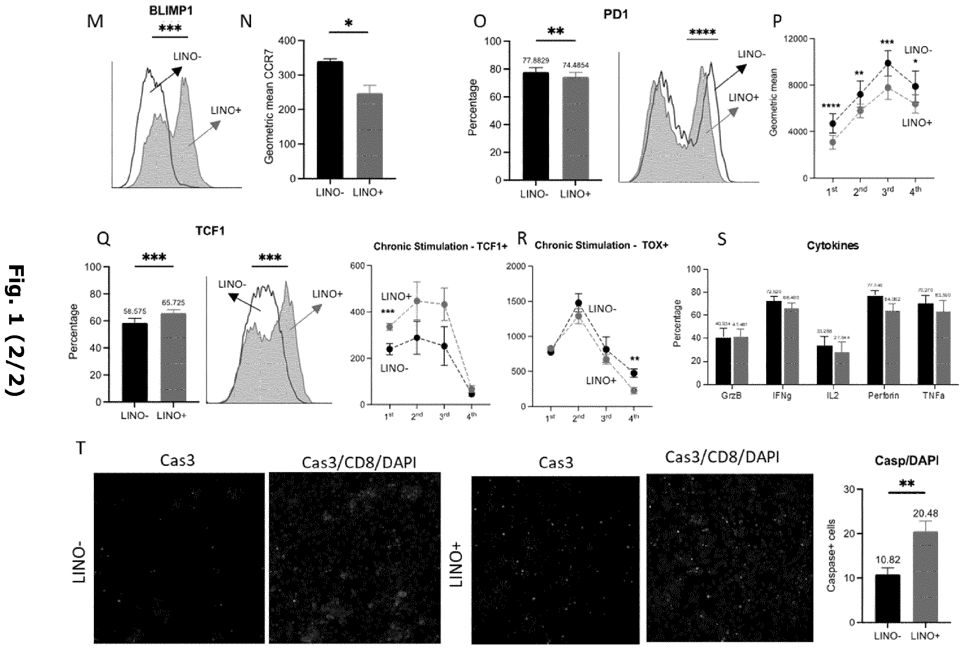

### Figure 1. LINO-treatment induces a memory-like phenotype in CD8+ OT1 cells whilst retaining effector features

The inventors isolated CD8+ T cells from OT1 mice by negative selection using magnetic beads and activated them in the presence or absence of LINO as indicated in A. After 48 hours in culture, inventors performed CFSE staining **(B)** and manual cell counting using a Neubauer chamber **(C).**

Immunophenotype of LINO-treated cells by flow cytometry using CD62L **(D),** CD127 **(E),** CXCR3 **(F)** led to the identification of a T Central Memory population **(G);** characterized by high expression of transcription factor EOMES **(H)** and decreased Tbet **(I). (J)** Light microscopy morphological assessment of cluster size and number in LINO-treated cells and controls after 48 hours of culture was in line with **(K)** quantification of cell relative granulocity/internal complexity by SSC and increased CD44 percentages in LINO-treated cells **(L).** Representative histogram for the quantification of mean fluorescence intensity (MFI) of transcription factor Blimp1 **(M)** and surface marker CCR7 **(N).** PD1 expression was assessed as percentage and MFI after 48 hours in culture (**O**) and upon chronic stimulation **(P).** Chronic stimulation experiment was performed by continually supplying cells with OVA peptide in the presence or absence of LINO. Cell phenotype was assessed by flow cytometry every 48 hours after 1st, 2^{nd}, 3^{rd} and 4^{th} stimulation cycles. Likewise, inventors assessed the expression of transcription factors TCF1 **(Q)** and TOX **(R).** Effector cytokine production was assessed by intracellular staining **(S)** and by immunofluorescence with an antigen specific in vitro killing assay **(T)** where inventors quantified Caspase 3 production by tumour cells after 24 hours of co-culture with LINO-treated or control OT1 cells. Data are presented as mean ± SEM. Paired student T-test was used to compare groups. ^{∗} p<0.05, ^{∗∗} p<0.01, ^{∗∗∗} p<0.001, ^{∗∗∗∗} p<0.0001, n, not significant.

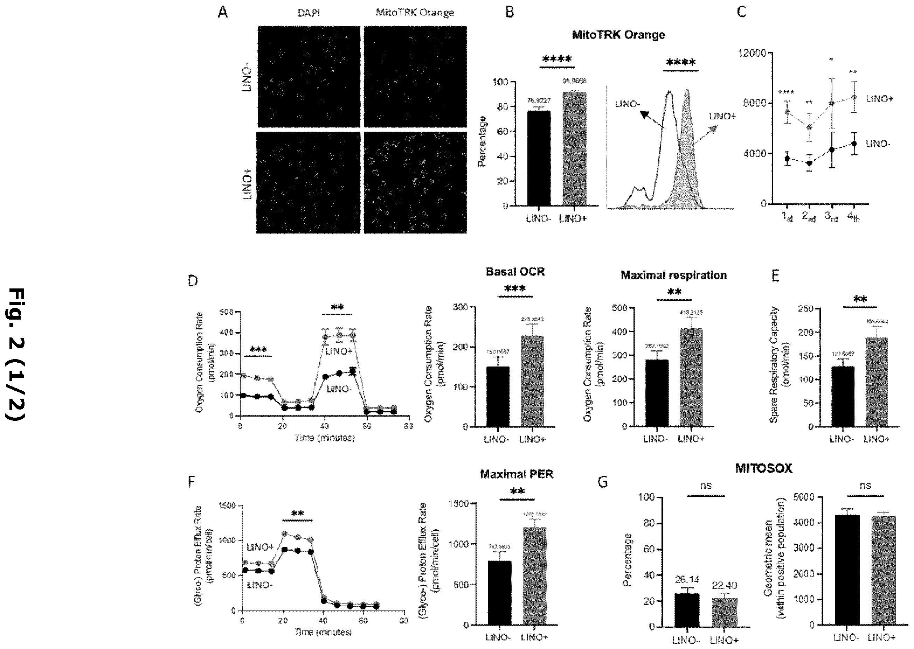

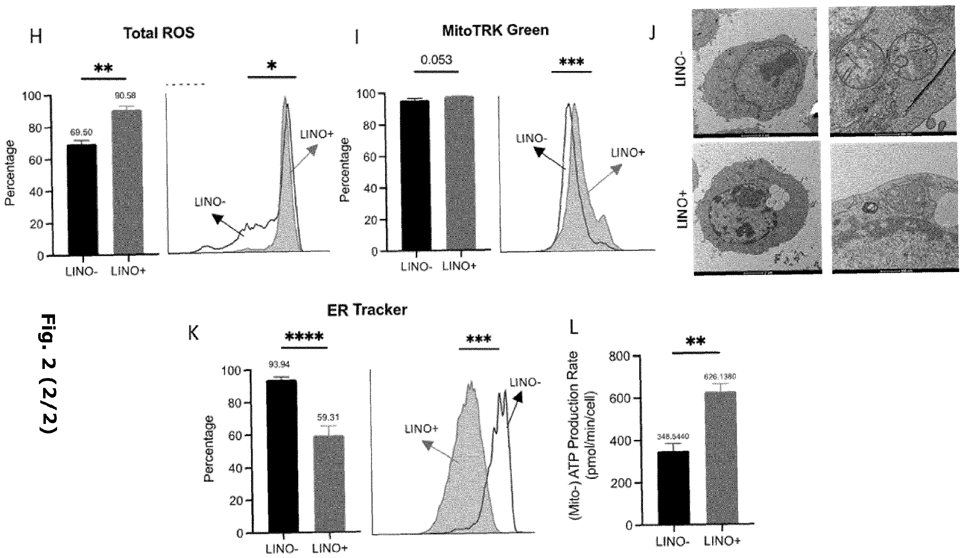

### Figure 2. LINO-treated CD8+ T cells display higher metabolic fitness driven by mitochondrial capacity

To assess the metabolic landscape of OT1 CD8+ T cells inventors used MitoTRK Orange staining by immunofluorescence **(A)** and flow cytometry **(B).** Quantification in flow cytometry was performed both by percentage (left panel) and MFI (right panel). Inventors also assessed MitoTRK Orange in a chronic stimulation setting as previously described and quantified the MFI every 48 hours after 1st, 2^{nd}, 3^{rd} and 4^{th} stimulation cycles **(C).** MitoStress Test assay was performed at the SeaHorse extracellular analyzer (n=12). Oxygen Comsumption Rate (OCR) was monitored in real time after injection of each compound to build the kinetic profile of LINO-treated and control cells and quantify basal and maximal respiration **(D).** Spare Respiratory Capacity (SRC) was calculated as the difference between maximal and basal respiration rates **(D).** Glycolytic Rate assay was also performed exploiting the SeaHorse platform (n=9) to obtain a glycolytic profile and quantify the maximal proton efflux rate (PER) which is directly proportional to glycolytic performance upon stress **(F).** Inventors assessed mitochondrial superoxide production levels by MitoSOX staining **(G)** and also quantified total ROS production as percentages and MFI **(H).** To assess mitochondrial mass and structure inventors used MitoTRK Green staining (**I**) and transmission electron microscopy (**J**). **(K)** Sulfonylurea receptors of ATP-sensitive K+ channels (K(ATP)) were used as a surrogate for endoplasmic reticulum (ER) tracker, and the mitochondrial ATP production rate **(L)** was calculated following the formula: OCR_{ATP} (pmol O2/min) = OCR (pmol O2/min) - OCR_{Oligo} (pmol O2/min).

Data are presented as mean ± SEM. Paired student T-test was used to compare groups. ^{∗} p<0.05, ^{∗∗} p<0.01, ^{∗∗∗} p<0.001, ^{∗∗∗∗} p<0.0001, n, not significant.

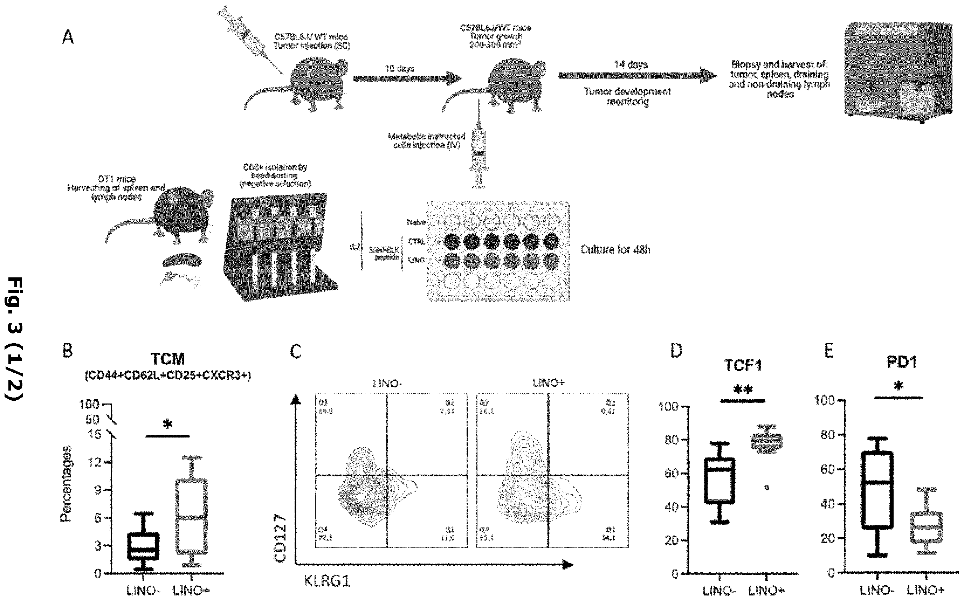

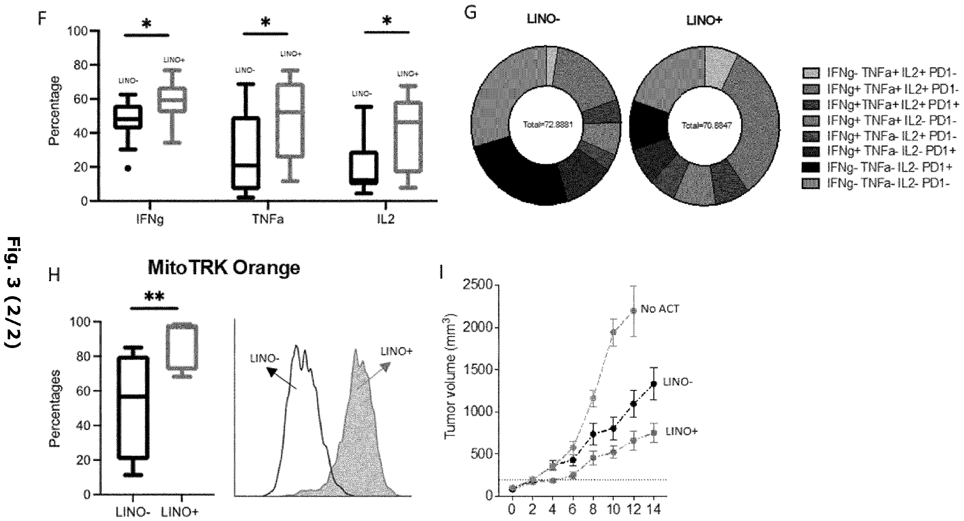

### Figure 3. ACT of LINO-treated cells results in tumour control in an antigen specific melanoma model

**(A)** In vivo experiments were performed using our antigen-specific melanoma cell line. Metabolically instructed OT1 cells were injected intravenously into tumour-bearing mice and tumour development was monitored every 48 hours for up to 14 days. At the end time-point spleen, draining and non draining lymph nodes and tumours were harvested and prepared for flow cytometry assays (No ACT=14 mice, LINO- ACT control group= 12 mice, LINO+ ACT= 14 mice).
**(B)** Assessment of differentiation status of adoptively transferred LINO-treated cells shows an increase in Tcm population, with higher expression of CD127 and lower levels of KLRG1 **(C).**

LINO-treated cells were characterized by increased percentages cells expressing transcription factor TCF1 **(D)** and decreased expression of exhaustion marker PD1 **(E). (F)** Effector cytokine production was measured by intracellular staining and poly-functionality analysis was performed to identify effector sub-populations distribution among treatments **(G). (H)** Metabolic fitness was assessed by MitoTRK staining and quantified as percentage of positive cells and MFI. **(I)** Continuous monitoring of tumour growth allowed the construction of a growth curve using the formula: Tumour volume= length x width²/2 where length represents the largest tumour diameter and width represents the perpendicular tumour diameter. Data are presented as mean ± SEM. Un-paired student T-test was used to compare groups. ^{∗} p<0.05, ^{∗∗} p<0.01, ^{∗∗∗} p<0.001, ^{∗∗∗∗} p<0.0001, n, not significant.

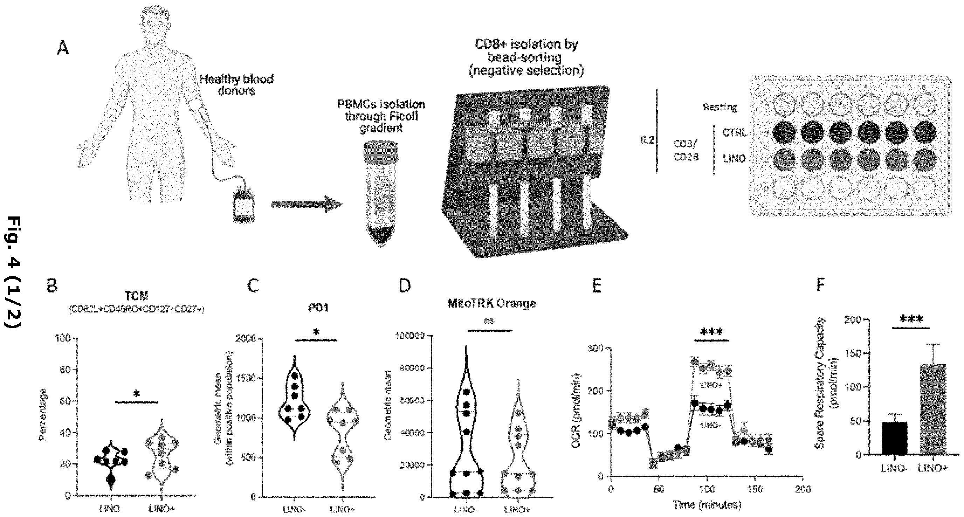

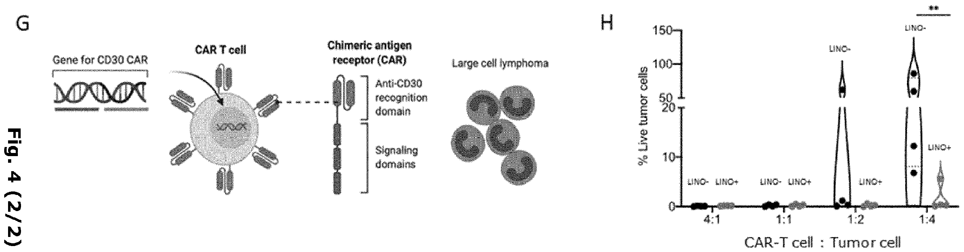

### Figure 4. LINO-induced phenotype is reproducible in human T cells and increases CAR-T cell cytotoxic activity

**(A)** Peripheral blood mononuclear cells (PBMCs) were isolated from samples obtained from healthy donors or anonymous buffy coats of healthy donors using Ficoll density separation. CD8+ T cells were obtained from PBMCs by negative magnetic and polyclonally. Cells were cultured for 48 hours in the presence or absence of LINO and their phenotype was assessed by flow cytometry and the extracellular flux analyzer SeaHorse.

Our differentiation flow cytometry panel showed human CD8+ T cells increase their pool of central memory population when treated with linoleic acid **(B). (C)** Quantification of PD1 expression was made using the MFI values. Metabolic characterization was performed using MitoTRK Orange **(D)** and the MitoStress Test assay from SeaHorse (n=5 healthy donors, **E).** Inventors also calculated the spare respiratory capacity (SRC) following the already described formula **(F).** Transduction of human CD8+ T cells with the Chimeric Antigen Receptor (CAR) for CD30 was performed by the Laboratory for Cell and Gene Therapy associated to University Children's Hospital of Würzburg. Inventors chose this antigen because it is expressed by non-Hodgkin's lymphomas and, in particular by large cell lymphoma which has a very low response rate to available treatments **(G). (H)** In vitro killing assay was performed using tumour cells expressing CD30 and CAR-T cells transduced in the presence or absence of LINO in the media (n= 4 healthy donors). Data are presented as mean ± SEM. Paired (B, C, D, E and F) and un-paired (H) student T-test was used to compare groups. ^{∗} p<0.05, ^{∗∗} p<0.01, ^{∗∗∗} p<0.001, ^{∗∗∗∗} p<0.0001, n, not significant.

### Examples

### MATERIALS AND METHODS

### CD8+ OT1 cell isolation and activation

Spleen and lymph nodes (inguinal and axillary) are harvested from OT1 mice (purchased from Charles River) under sterile conditions and mechanically dissociated into single cell suspension using a 40 micrometers cell strainer. Red blood cell lysis is performed using 1mL ACK buffer for 2 min in continuous but gentle agitation. After 2 minutes, ACK is stopped using 10x volumes of complete RPMI media and washed at 1500 rpm for 5 min. Cells are then counted and sorted using negative selection beads sorting and following the manufacturer's instructions (Miltenyi, CAT 130-104-075). After sorting, cells are counted and resuspended at 1x10^6 cells/mL in complete RPMI media (10% FBS, 1% NaPy, 1% Glutamine, 1% Pen/Strep, 0,1% beta-mercaptoethanol) supplemented with 100U/mL IL2 and 2 micrograms/mL of SIINFELK peptide (from Primmbiotech). Cells are further treated with BSA, palmitic acid (both from Agilent Technologies, CAT number 102720-100, 100 microL/mL); linoleic acid or oleic acid (both from Merk Life Sciences L9530-5ML and O3008-5ML respectively; 30 microL/mL). Final concentration for all lipids was stablished at 100 microMolar.

### Immunophenotype and flow cytometry

Characterization of the activation and differentiation profile of OT1 cells was performed by flow cytometry following standard protocols. Briefly, for staining of surface markers 2×10⁵ - 1×10⁶ cells were harvested at the specified post-activation time points, washed with PBS, resuspended in FACS buffer (PBS, 1% FBS, EDTA 2mM) and stained for 20 minutes at 4C. Intracellular staining was performed following a 4 hours incubation with complete RPMI and 1:1000 dilution of GlogiPlug (BD, 555029) in the presence or absence of a secondary stimulation with 5 micrograms/mL SIINFELK peptide (from Primmbiotech) at 37C. Following surface staining, cells were fixed and permeabilized using Foxp3 Transcription Factor Staining Buffer Set (eBiosciences, 00-5523-00) following manufacture's instruction. Intracellular staining was then performed in permeabilization buffer for 1 hour at 4C. CFSE (C34554); MitoTRK Orange (M7512) and Green (M7514); MitoSOX (M36008); and total ROS (88-5930-74) reagents were all acquired from ThermoFisher and performed as per manufacturer's instructions. All stainings were washed with PBS and acquired using a BD FACS Celesta (Flow Cytometry Facility IEO Campus).

### Immunofluorescence and confocal microscopy

Experiments with fixed cells were performed on a LEICA DMi8 (inverted) microscope (Microscopy core facility IFOM-IEO Campus) with a 63x oil- immersion objective. Cells were allowed to attach by gravity on culture slides (BD Bioscience) coated with poly-d-lysine for 40 min. Cells were pre-stained with MitoTRK Orange and a fixable viability dye and then fixed with 4% paraformaldehyde and permeabilized with 0.1% Triton X-100. Cells were stained with primary antibodies against Caspase 3 (Cell Signaling, 9661) overnight at 4C, labeled with the appropriate secondary antibodies, and counterstained with DAPI for 2 hours at room temperature.

### Metabolic assays

A Seahorse XF-96e extracellular flux analyzer (Seahorse Bioscience, Agilent) was used to determine the metabolic profile of cells. OT1 CD8+ T cells were plated (2×10⁵ cells/well) onto cell culture microplates coated with poly-lysine. Mitochondrial perturbation experiments were carried out using the MitoStress Test Kit (Agilent, 103015-100) by sequential addition of oligomycin (1 mM), FCCP (2 mM), and rotenone (1 mM). Oxygen consumption rates (OCR, pmol/min), proton efflux rate (PER, pmol/min), and extracellular acidification rates (ECAR, mpH/min) were monitored in real time after injection of each compound.

### Electron microscopy

Transmission electron microscopy was performed at IRCCS Facility (San Raffaele Hospital). Cells were sequentially fixed in 0,1M Cacodilate pH 7.4 for 1 hour, followed by cacodylate buffer. Image analysis and quantification is currently ongoing.

### Adoptive cell transfer (ACT) experiments

In vivo experiments were performed using our antigen specific melanoma cell line B16-OVA that was maintained in culture in RPMI, 10%FCS, 1%PenStrep, 0,4mg G418 up to 5 passages prior injection. C57B6/J mice were subcutaneously injected with 2×10⁵ cells and tumour growth was monitored daily. ACT was performed intravenously using 5×10⁶ OT1 cells previously activated in the presence or absence of LINO as previously described. Tumours were inspected and measured daily to construct a growth curve using the formula: Tumour volume= length x width2/2, where length represents the largest tumour diameter and width represents the perpendicular tumour diameter. On the day of analysis spleen and tumour were collected from each mouse. Spleens were smashed and total splenocytes were isolated after performing red cell lysis with a home-made isotonic solution. Tumours were collected, weighted and digested in RPMI 10% FCS 1%PS Collagenase IV (1mg/ml) and DNAse (100µg/ml) for 20 min at 37C. After digestion, approximately one million cells isolated from tumour or spleen were isolated and stained with for flow cytometry analysis.

### Isolation and culture of primary human T lymphocytes

Peripheral blood mononuclear cells (PBMCs) were isolated from samples obtained from healthy donors or anonymous buffy coats (purchased as discarded material from the blood bank) of healthy donors using Ficoll density separation. CD8+ T cells were obtained from PBMCs by negative magnetic selection (Miltenyi Biotec). T lymphocytes were activated with immobilized Anti-human CD3 (2 µg/ml, Invitrogen) and anti-CD28 (2 µg/ml, Invitrogen) antibodies and then expanded in complete RPMI 1640 medium (10% FBS, 1% NaPy, 1% Glutamine, 1% Pen/Strep) supplemented with 100 U/mL IL2. Cells were cultured for 48 hours in the presence or absence of LINO. Resting cells were kept as control with no further activation stimulus provided.

### CAR-T cell engineering and GMP protocols

Transduction of Chimeric Antigen Receptor T cells (CAR-T cells) was performed at Laboratory for Cell and Gene Therapy associated to University Children's Hospital of Würzburg. HPSE cDNA (accession number NM_006665) was cloned into the SFG retroviral backbone that also encodes the GFP (SFG.HPSE(I)GFP). Inventors used the already available construct for the GD2-specific CAR containing the CD28, OX40 and ζ endodomains. Inventors then generated a bicistronic vector to co-express the HPSE and CAR-CD30 using an IRES (SFG.CAR(I)HPSE). The retroviral vector encoding the fusion protein GFP-firefly luciferase (GFP.FFLuc) for in vivo imaging of T cells was also available at the lab. A specific inhibitor of HPSE, Roneparstat (SST0001) (a chemically modified heparin 100Na, Ro-H, property of Sigma-tau Research Switzerland S.A.) (3 µg ml), was added to the media during the virus preparation to increase its titer. Activated T lymphocytes (i.e. the CD4 and CD8 T cells from healthy donors PBMCs or anonymous buffy coats of the previous paragraph after 48h of activation) were then transduced with retroviral supernatants using retronectin-coated plates (Takara Bio Inc., Shiga, Japan). After removal from the retronectin plates, T cell lines were maintained in complete T cell medium in a humidified atmosphere containing 5% CO2 at 37 °C in the presence of IL-2 (50 U mL) for 2 weeks.

### RESULTS

### 1. LINO-treatment induces a memory-like phenotype in CD8+ OT1 cells whilst retaining effector features

The metabolic fitness of Tumour infiltrating lymphocytes (TILs) plays an important role in the exertion of effector functions and thus, tumour control. To address the question of whether lipids can induce metabolic changes in CD8+ T cells, inventors first evaluated the effect of linoleic acid (LINO) in the activation status of murine OT1 CD8+ T cells. Inventors harvested spleen and lymph nodes from OT1 mice and isolated CD8+ T cells by negative sorting. Cells were then activated in the presence or absence of LINO for 48 hours and immunophenotype was assessed by multicolour flow cytometry (figure 1.A). After 48h in culture, CFSE staining did not show any difference in proliferative potential between treated and un-treated cells (figure 1.B). However, LINO treatment did lead to an increased fold change in cell number (n=36, figure 1.C), suggesting a slight but significantly increased proliferation capacity with respect to control cells. A comprehensive differentiation panel performed by multicolour flow cytometry showed LINO-treated cells are characterized by increased expression of canonical memory surface markers CD62L (figure 1.D) and CD127 (figures 1.E). Further characterization on the memory compartment of LINO-treated cells confirmed the co-expression of CD25 and CXCR3 (figure IF), allowing the identification of a slightly but significantly increased T central memory population (figure 1.G). Together with augmented expression of transcription factor eomesodermin (EOMES, figure 1.H) and decreased levels of T-bet (figure 1.I), these data suggest LINO-treated cells have the potential to become long-lived memory cells with high cellular fitness (Kaech, 2003), while retaining an early differentiation state (Intlekoger, 2005). Morphologic assessment of LINO-treated cells revealed an increase in cluster size and number (figure 1.J) as well as a significant increase in SSC values measured by flow cytometry (figure 1.K). Together, these observations indicate an enhanced activation status of LINO-treated cells. In fact, a deep characterization of canonical activation markers, showed a slight but reproducible increase in CD44 percentages in LINO-treated cells (n=45, figure 1.L), while all the other assessed activation markers were not modulated. Inventors also assessed the expression levels of transcription factor Blimp1 by quantification of the mean fluorescent intensity (MFI). Results revealed LINO-treated cells display a 4-fold increase in Blimp1 expression with respect to controls (n=15, figure 1.M) leading to the believe LINO treatment might increase the efficiency in recall response of OT1 CD8+ T cells (Kallies, 2009). In line with this data, expression levels of CCR7 were downregulated in LINO-treated cells (figure 1.N), further supporting the theory of an increased capability to migrate towards inflammation centres (Forster, 2008). Surprisingly, and even though Blimp1 expression has been associated with upregulation of inhibitory receptors in chronic viral infection (Shin, 2009), PD1 levels were significantly decreased in LINO-treated cells (n=45) both as percentage of positive cells and MFI quantification (figure 1.O). To further investigate PD1 modulation by LINO treatment and better mimic the persistent antigen stimulation found in the tumour microenvironment, inventors performed a chronic stimulation assay with continuous supply of LINO in the cell media. Our results show a significant and sustained downregulation of PD1 expression in LINO-treated cells (n=10, figure 1.P), suggesting a reduced likelihood of exhaustion induction by PDL1 ligand in the tumour site and, in general, a less exhausted phenotype of LINO-treated cells when subjected to continuous stimulation. In line with these results, inventors also observed a significant increase in the expression of TCF1 (figure 1.Q), a transcription factor described to drive a progenitor-like effector versus exhausted phenotype in T cells during chronic viral infection (Chen, 2019); and decreased expression of TOX (figure 1.R) suggesting LINO-treated cells might have an increased cytotoxic capacity with increased production of effector cytokines (Seo, 2019). In order to test this theory, inventors perform intracellular cytokine staining and noticed LINO-treated cells retain the same cytokine production capabilities as non-treated control cells (n=10, figure 1.S). However, the cytotoxic activity of LINO-treated cells in an in-vitro killing assay, was able to induce a 2-fold increase in tumour cell death (n=6, figure 1.T). Altogether, these data demonstrate LINO-treated cells hold an increased killing capacity while still retaining a memory-like phenotype and significantly reduced levels of PD1 expression.

### 2. LINO-treated CD8+ T cells display higher metabolic fitness driven by mitochondrial capacity

It has been widely reported that metabolic constraints found in the tumour microenvironment have a deep impact in T cell differentiation patterns and effector functions. In fact, every single differentiation state is characterized by the engagement of specific metabolic pathways. Upon antigen encounter, a naive T cell is activated towards an effector phenotype that relies mainly on glycolysis and requires a significant increase in nutrient uptake (Pearce, 2013). Once the infection has been cleared, a small sub-population of effector T cells differentiate to a memory T cell population, responsible for immunosurveillance with a metabolism mostly based on oxidative phosphorylation (OXPHOS) and boosted by increased mitochondrial mass (Pearce, 2013). In view of the tight connection between T cell metabolism and their anti-tumour capacity, inventors decided to characterize the metabolic landscape driven by LINO treatment. Inventors assessed mitochondrial activity using MitoTRK Orange by immunofluorescence (n=6, figure 2.A) and flow cytometry (n=45). The results show a significant increase in MitoTRK Orange on LINO-treated cells with respect to controls, not only in percentage of positive cells but also in MFI (figure 2.B). Furthermore, LINO-treated cells exhibited a significant and sustained increase in mitochondrial membrane's potential when submitted to our chronic stimulation setting (figure 2.C), suggesting a highly improved metabolic fitness, which might confer them a selective advantage in the metabolic hostile TME. To further characterize the metabolic response of LINO-treated cells to stress conditions, inventors exploited the extracellular flux analyser SeaHorse. In line with MitoTRK results and the stablished memory-like phenotype, LINO-treated cells showed a basal metabolism with significant increased OXPHOS, measured as oxygen consumption rate (OCR, figure 2.D). But most importantly, upon mitochondrial complexes disruption, LINO-treated cells showed a striking increase in maximal respiration levels and Spare Respiratory Capacity (SRC, figure 1.E), in line with their memory phenotype and further confirming their capability to adapt to stress conditions. Additionally, glycolytic rate assays showed a high increase in the maximal Proton Efflux Rate (PER, figure 2.F); suggesting a bioenergetic profile capable of sustaining effector functions upon stress. Taken together, these results support a highly increased metabolic fitness of LINO-treated cells that might be responsible for sustaining effector functions as well as a memory progenitor phenotype. Surprisingly, when assessing mitochondrial superoxide levels by MitoSOX staining, inventors found no differences with respect to controls (n=5, figure 2.G), while total Reactive Oxygen Species (ROS) levels were significantly increased in LINO-treated cells both in percentage (≈20%) and MFI (n=5, figure 2.H). These results show that, regardless of the outstanding increase in MitoTRK Orange, mitochondrial superoxide does not differentially influence total ROS levels. Inventors then wonder whether LINO treatment could affect mitochondrial structure and decided to assess organelle structure by Transmission Electron Microscopy (TEM). Regardless of the noticeable increase in MitoTRK Green staining (n=26, figure 2.I), TEM results didn't show any significant difference in the number, area or shape of mitochondria of LINO-treated cells with respect to controls (n=3). Nevertheless, structural analysis showed differences in the distribution and length of the endoplasmic reticulum (ER, figure 2.J) as well as differences in the number of mitochondria-ER contact sites (data not shown-quantification analysis in progress), that inventors aim to quantify and investigate further. In light of these findings, inventors decided to initiate our characterization of the ER by assessing ER Tracker Green (BODIPY Glibenclamide) staining by flow cytometry. The ER Tracker dye; that binds to the sulfonylurea receptors of ATP-sensitive K+ channels (K(ATP)); was strikingly decreased both as percentage of positive cells (≈30%) and MFI (3-fold) in LINO-treated cells (n=9, figure 2.K). Considering K(ATP) channels open as cellular ATP levels fall (Almanza, 2019); its low levels are in line with the significant (2-fold) increase in mitochondrial-produced ATP measured at the SeaHorse platform (figure 2.L). Moreover, inhibition of K(ATP) channels leads to the ER depolarization and increased intracellular Ca+ levels in beta-cells (Tinker, 2018), further suggesting an implication of Ca+ influx in the LINO-induced metabolism. This hypothesis was confirmed by a preliminary experiment indicating the ability of LINO treatment to positively affect T cell antigen sensitivity and increase Ca+ signalling. In all, these data show LINO-treated cells are characterized by (1) a significantly increased metabolic fitness mainly based in OXPHOS, with a (2) high SRC and the (3) potential to engage in glycolysis upon stress conditions. These metabolic features further confirm a LINO-induced memory-like phenotype, and highlight their potential to exert effector functions when submitted to stress.

### 3. ACT of LINO-treated cells results in tumour control in an antigen specific melanoma model

Memory T cells are reported to have higher metabolic fitness, long term persistence, increased recall response, and high transcriptional plasticity (Romero et all. 2019; Martin and Badovinac, 2018); making them the perfect candidate for ACT treatment. Following our extensive characterization of the LINO-induced phenotype, inventors hypothesized its memory-like phenotype, reduced expression of PD1, and increased metabolic fitness could represent a great advantage in the TME and achieve tumour control. Thus, inventors moved forward with the in vivo evaluation of LINO-treated cells as ACT product in an antigen specific melanoma murine model. Inventors harvested spleen and lymph nodes from OT1 mice, isolated CD8+ T cells, activated them in the presence or absence of LINO and kept them in culture for 48h to produce our metabolically instructed T cells. They were infused in tumour bearing mice intravenously and then inventors monitored tumour progression, for up to 14 days when inventors performed flow cytometry assays to characterize the exogenous T cell phenotype (figure 3.A). 14 days post-infusion, LINO-treated T cells maintained their memory-like phenotype characterized by co-expression of CD44, CD62L, CD25 and CXCR3 (figure 3.B). Together with this data, an enrichment of the CD127+KLRG1- population (figure 3.C) indicates LINO-treated cells retain their memory-like phenotype while showing low senescence levels. Inventors also observed an increased expression of transcription factor TCF1 (figure 3.D) and a significant decrease in PD1 positive cells (figure 3.E), which gives a clear indication of reduced exhaustion features in LINO-treated exogenous cells. Altogether, and in line with our in vitro results, these data suggest LINO-treated cells are capable of long-term survival in the TME giving a noticeable advantage with respect to control ACT. Evaluation of killing capacity was performed by intracellular staining of effector cytokines, and the results showed a significantly increased percentage of LINO-treated cells producing IFNγ, TNFα and IL2 (figure 3.F), making them more cytotoxic than control cells. A deeper insight into poly-functionality in adoptively transferred OT1 cells, allowed the identification of 8 different sub-populations (figure 3.G). In line with our initial observations, control cells were represented mostly (>50%) by IFNγ-TNFα-IL2-PD1+ exhausted T cells (in black) and IFNγ-TNFα-IL2-PD1- anergic-like cells (in dark grey). On the contrary, LINO-treated cells were characterized by a significant increase in the IFNγ+TNFα+IL2+PD1- T cell population (in light blue), further confirming the predominance of a fully functional cytotoxic machinery. Interestingly, LINO-treated cells also showed a 2-fold increase in the IFNγ+TNFα+IL2+PD1+ population (in dark blue) with respect to control cells; suggesting they hold the capability to sustain high cytotoxicity regardless of PD1 expression. Moreover, inventors performed MitoTRK Orange staining, and surprisingly LINO-treated cells kept their characteristic increased mitochondrial potential 14 days post-infusion both as percentage of positive cells and MFI quantification (figure 3.H); confirming the retention of the metabolic fitness induced by the in vitro LINO-treatment. Taken together, and in line with the increased memory-like phenotype, the decreased levels of PD1 expression, and the significant boost in metabolic fitness and effector cytokine production, LINO-treated ACT therapy was able to significantly reduce tumour growth in our antigen-specific melanoma murine model (figure 3.I). Of notice, the effect on tumour control seems to initiate at an early time point, since inventors observe a highly significant difference just 4 days post ACT infusion, when the CTRL and non-treated groups are still the same volume. This observation recapitulates the striking increase in Blimp 1 expression (figure 1.M) and the downregulation of CCR7 (figure 1.N) observed in vitro; proposing a high recall capacity of LINO-treated cells that allow them to migrate faster towards the tumour site. In summary, our data identify linoleic acid (LINO) as a LCFA able to drive the metabolic rewiring of CD8+ T cells towards a T Central memory-like phenotype with high persistence potential, increased mitochondrial fitness, and significant tumour killing capabilities.

### 4. LINO-induced phenotype is reproducible in human T cells and increases CAR-T cell cytotoxic activity

Considering the incredible potential of T cell metabolic rewiring for ACT treatment in the clinic, inventors moved forward with the characterization of the LINO-induced phenotype using human CD8+ T cells. Inventors used negative selection sorting to purify CD8+ T cells from healthy donors PBMCs or anonymous buffy coats of healthy donors and activated them in a polyclonal setup in the presence or absence or LINO in the media (figure 4.A). 48 hours post-activation, cells were harvested and analysed by multicolour flow cytometry and the results mostly complemented the phenotype inventors found in mouse. Inventors found a 1.3-fold increase in T central memory population, defined as CD62L+CD45RO+CD127+CD27+ (figure 4.B) and characterized by a significant decrease in PD1 expression measured as MFI (figure 4.C). When assessing the metabolic landscape of LINO-treated human CD8+ T cells inventors noticed no significant differences in MitoTRK Orange staining (figure 4.D). However, assessment of the OCR using the SeaHorse platform showed LINO-treated cells had a significant increased OXPHOS activity upon mitochondrial disruption. Furthermore, LINO-treated cells retained their characteristically high SRC (figure 4.F); suggesting the treatment does indeed increase metabolic fitness upon stress conditions also in human CD8+ T cells. Altogether, these promising results suggest LINO-treatment could be proposed for the manufacturing of ACT products. Thus, inventors decided to set up a clinical protocol following GMP practices and follow the LINO-induced phenotype on human Chimeric Antigen Receptor (CAR) T cells. CAR-T cells have proved to be very efficient in mediating tumour control by using tumour specific antigens to construct the chimeric antigen receptor. Inventors transduced our T cells with a CAR for CD30, that is an antigen expressed by non-Hodgkin's lymphomas and, in particular by large cell lymphoma which has a very low response rate to available treatments (figure 4.G). In order to test whether LINO treatment could increase the metabolic fitness of CAR-T cells, inventors setup an in vitro killing assay using tumour cells expressing CD30 and CAR-T cultured in the presence or absence of linoleic acid. Results show a very strong killing efficiency by both CTRL cells and LINO-treated cells when combined in a favourable ratio (4 T cells vs 1 tumour cells or 1 T cell vs 1 tumour cell). Nevertheless, when put into a high numerical disadvantage: 1 CAR-T vs 4 tumour cells, LINO-treated CAR-T cells were able to retain their cytotoxic capabilities (figure 4.G). These data suggest a superior effector function of LINO-treated CAR-T cells and it is further proof that LINO-driven metabolic rewiring could potentially increase the efficiency of immunotherapy approaches.

**TABLE**

| | | | |
|---|---|---|---|
| Enhanced metabolic fitness | MitoTRK Orange | ⇑ (1.6-fold change) | |
| | MitoTRK Green | ⇑ (1.4-fold change) | |
| | SRC | ⇑ 1.5-fold change | |
| | Basal OCR | ⇑ 1.5-fold change | |
| | Maximal PER | ⇑ 1.5-fold change | |
| | Mito-ATP production rate | ⇑ 1.8-fold change | |

| | | **Mouse** | **Human** |
|---|---|---|---|
| Enriched memory population | TCM | CD44+CD62L+CD25+CXCR3+ ⇑ 1.2-fold change | CD45RO+CD62L+CD127+CD27+ ⇑ 1.6-fold change |
| | TCF1 | ⇑ 1.2-fold change | |
| | Blimp1 | ⇑ 1.4-fold change | |
| Reduced expression of exhaustion markers | PD1 | ⇓ 1.3-fold change | |

| | | **Mouse** | **Human** |
|---|---|---|---|
| Polyfunctionality | Co-expression of IFNγ, TNFα and IL2 | *In vivo:* ⇑ ft 1.9-fold change | |
| Cytotoxicity | *In vitro* killing assay | ⇑ Caspase+ tumor cells 2-fold change | ⇓ Live tumor cells (20-fold change) |

Table summarizing the different features specifically induced by LA treatment. In details, **metabolic fitness** was quantified by assessing mitochondrial potential (MitoTRK Orange) and mass (MitoTRK Green) together with their energetic profile in terms of SRC, OCR, PER and ATP production; **memory phenotype** was measured based on the expression of several memory associated markers and **reduced exhaustion** was assessed by the downregulation of PD1 which was associated with increased polyfunctionality and cytotoxic ability.

### REFERENCES

A. Almanza, A. Carlesso, C. Chintha, S. Creedican, D. Doultsinos, B. Leuzzi, A. Lus, N. McCarthy, L. Montibeller, S. More, A. Papaioannou, F. Puschel, M.L. Sassano, J. Skoko, P. Agostinis, J. Belleroche, L.A. Eriksson, S. Fulda, A.M. Gorman, A. Kozlov, C. Munoz-Pinedo, M. Rehm, E. Chevet, S. Healy, and A. Samali. "Endoplasmic reticulum stress signalling - from basic mechanisms to clinical applications". The FEBS Journal 286 (2019) 241-278.
R. Förster, A.C. Davalos-Misslitz and A. Rot. "CCR7 and its ligands: balancing immunity and tolerance". Nature Immunology doi:10.1038/nri2297 Published online 1 April 2008.
A.M. Intlekofer, N. Takemoto, E.J. Wherry, S.A. Longworth, J.T. Northrup, V.R. Palanivel, A.C. Mullen, C.R. Gasink, S.M. Kaech, J.D Miller, L. Gapin, K. Ryan, A.P Russ, T. Lindsten, J.S. Orange, A.W. Goldrath, R. Ahmed and S.L. Reiner. "Effector and memory CD8+ T cell fate coupled by T-bet and eomesodermin". Nature Immunology, doi:10.1038/nil268 Published online 6 November 2005.
S.M Kaech, J.T. Tan, E.J. Wherry, B.T. Konieczny, C.D. Surh and R. Ahmed. "Selective expression of the interleukin 7 receptor identifies effector CD8 T cells that give rise to long-lived memory cells". Nature Immunology Published online 16 November 2003; doi:10.1038/ni1009.
A. Kallies, A. Xin, G.T. Belz and S.L. Nutt. "Blimp-1 Transcription Factor Is Required for the Differentiation of Effector CD8+ T Cells and Memory Responses". Immunity 31, 283-295, August 21,2009.
M.D Martin and V.P. Badovinac. "Defining Memory CD8 T Cell". Front Immunol 2018 Nov 20;9:2692. doi: 10.3389/fimmu.2018.02692.
E.L. Pearce, M.C. Poffenbergerchih-Hao, C. Russel and G. Jones. "Fueling Immunity: Insights into Metabolism and Lymphocyte Function". Science. 11 Oct 2013, Vol 342, Issue 6155, DOI: 10.1126/science.1242454
P. Romero, A. Zippelius, I. Kurth, M.J. Pittet, C. Touvrey, E.M. Iancu, P. Corthesy, E. Devevre, D.E. Speiser and N. Rufer. "our Functionally Distinct Populations of Human Effector-Memory CD8+ T Lymphocytes". J Immunol April 1, 2007, 178 (7) 4112-4119; DOI: https://doi.org/10.4049/jimmunol.178.7.4112.
H. Seo, J. Chen, E. Gonzalez-Avalos, D. Samaniego-Castruita, A. Das, YH. Wang, I.F.. Lopez-Moyado, R.O. Georges, W. Zhang, A. Onodera, C.J. Wu, L.F. Lu, PG. Hogan, A. Bhandoola, and A. Rao. "TOX and TOX2 transcription factors cooperate with NR4A transcription factors to impose CD8+ T cell exhaustion". Proc. Natl Acad. Sci. USA 116, 12410-12415 (2019)
H. Shin, S.D. Blackburn, A.M. Intlekofer, C. Kao, J.M. Angelosanto, S.L. Reiner and E.J. Wherry. "A Role for the Transcriptional Repressor Blimp-1 in CD8+ T Cell Exhaustion during Chronic Viral Infection". Immunity 31, 309-320, August 21, 2009.
A. Tinker, Q. Aziz, Y. Li, and M. Specterman. "ATP-Sensitive Potassium Channels and Their Physiological and Pathophysiological Roles". Published online, October 2018 (comprehensivephysiology.com) DOI: 10.1002/cphy.c170048.

## Claims

1. A method for obtaining a T cell therapeutic comprising the steps of:
i) isolating at least one population of T cells from a biological sample isolated from a subject,
ii) activating said at least one population of T cells,
iii) treating said at least one population of T cells with a long chain fatty acid (LCFA), preferably said long chain fatty acid being selected from: linoleic acid and oleic acid,
thereby obtaining the T cell therapeutic.

2. The method of claim 1, wherein the population of T cells of step i) is CD4+, CD8+ or Gamma delta (γδ) T cells
and/or wherein the method further comprises a step wherein the T cell therapeutic are expanded in the presence of the long chain fatty acid (LCFA), preferably said long chain fatty acid being selected from: linoleic acid and oleic acid.

3. The method of claim 1 or 2 wherein step ii) and iii) are performed at the same time.

4. The method of any one of previous claims, wherein the obtained T cells therapeutic have at least one of the following properties: higher mitochondrial fitness, potent functionality, memory-like phenotype, higher cytotoxicity, reduced exhaustion features, increased proliferation capacity with respect to control cells
and/or wherein the obtained T cell therapeutic are **characterized by** at least one of the following:
- increased positivity to Mitotracker,
- increase in maximal respiration levels and Spare Respiratory Capacity (SRC), Oxygen Comsumption Rate (OCR), proton efflux rate (PER) and ATP production,
- co-expression of CD44, CD62L, CD25 and CXCR3,
- an enrichment of CD127+KLRG1- population,
- increased expression of transcription factor TCF1,
- a decrease in PD1 positive cells,
- increased expression of canonical memory surface markers CD62L and CD127,
- increased percentage of cells producing IFNγ, TNFα and IL2,
- increase in the IFNγ+TNFα+IL2+PD1- T cell population,
- increased expression of transcription factor eomesodermin (EOMES),
- decreased levels of T-bet,
- increase in cluster size and number,
- increase in SSC values measured by flow cytometry,
- increase in CD44 percentages cell,
- increase in Blimp 1 expression,
- decreased expression of CCR7,
- decreased expression of TOX,
- increase in their pool of T central memory population, defined as CD62L+CD45RO+CD127+CD27+ and **characterized by** a significant decrease in PD1,
with respect to controls cells.

5. The method of any one of previous claims, further comprising introducing in said population of T cells a nucleic acid sequence encoding an exogenous gene, thereby producing an engineered T cell therapeutic, preferably wherein the exogenous gene encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen.

6. The method according to claim 5 wherein said antigen recognizing receptor is a T cell receptor (TCR) or a chimeric antigen receptor (CAR).

7. The method according to any one of claims 5-6 wherein said nucleic acid sequence is introduced by a vector, preferably a retroviral or lentiviral vector,
and/or wherein said nucleic acid sequence is placed at an endogenous gene locus of the T cell.

8. A T cell therapeutic or an engineered T cell therapeutic obtainable by the method of any one of previous claims.

9. An isolated T cell population **characterized by** at least one of the following:
- increased positivity to Mitotracker,
- increase in maximal respiration levels and Spare Respiratory Capacity (SRC), Oxygen Comsumption Rate (OCR), proton efflux rate (PER) and ATP production,
- co-expression of CD44, CD62L, CD25 and CXCR3,
- an enrichment of CD127+KLRG1- population,
- increased expression of transcription factor TCF1,
- a decrease in PD1 positive cells,
- increased expression of canonical memory surface markers CD62L and CD127,
- increased percentage of cells producing IFNγ, TNFα and IL2,
- increase in the IFNγ+TNFα+IL2+PD1- T cell population,
- increased expression of transcription factor eomesodermin (EOMES),
- decreased levels of T-bet,
- increase in cluster size and number,
- increase in SSC values measured by flow cytometry,
- increase in CD44 percentages cell,
- increase in Blimp1 expression,
- decreased expression of CCR7,
- decreased expression of TOX,
- increase in their pool of T central memory population, defined as CD62L+CD45RO+CD127+CD27+ and **characterized by** a significant decrease in PD1,
with respect to controls cells.

10. An isolated T cell **characterized by** being CD44+, CD62L+, CD25+, CXCR3+, CD127+ and KLRG1- and PD1-
and/or by being CD62L+CD45RO+CD127+CD27+
and/or by at least one of:
- increased positivity to Mitotracker,
- increase in maximal respiration levels and Spare Respiratory Capacity (SRC), Oxygen Comsumption Rate (OCR), proton efflux rate (PER) and ATP production,
- increased expression of transcription factor TCF1 and /or eomesodermin (EOMES) and/or Blimp1,
- being able to produce IFNγ, TNFα and IL2,
- decreased expression of T-bet, CCR7 and of TOX,
- increase in cluster size,
- increase in SSC values measured by flow cytometry
with respect to controls cells.

11. The isolated T cell population of claim 9 or the isolated T cell of claim 10 engineered to comprise a nucleic acid sequence encoding an exogenous gene wherein the exogenous gene preferably encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen.

12. The engineered isolated T cell population or the isolated T cell of claim 11 wherein said antigen recognizing receptor is a T cell receptor (TCR) or wherein said antigen recognizing receptor is a chimeric antigen receptor (CAR), and/or wherein said nucleic acid sequence is introduced by a vector and/or wherein said nucleic acid sequence is placed at an endogenous gene locus of the T cell.

13. A pharmaceutical composition comprising the T cell therapeutic or the engineered T cell therapeutic of claim 8, the isolated T cell population of claim 9, the isolated T cell of claim 10 or the engineered isolated T cell population or T cell of claim 11 or 12.

14. The T cell therapeutic or the engineered T cell therapeutic of claim 8, the isolated T cell population of claim 9, the isolated T cell of claim 10 or the engineered isolated T cell population or T cell of claim 11 or 12 or the pharmaceutical composition of claim 13 for use as a medicament, preferably for use in the treatment of tumors, more preferably of solid tumors and hematological tumors refractory to current therapies, more preferably melanoma, neuroblastoma, lymphoma and myeloma, more preferably for adoptive cell therapy.

15. Use of a long chain fatty acid, preferably linoleic acid or oleic acid, to increase the pool of central memory population in an isolated population of T cells
and/or to obtain an isolated T cell therapeutic and/or engineered T cell therapeutic and/or T cell population and/or a T cell as defined in any one of claims 4-5 or 9-12.
